# EUROPEAN PATENT SPECIFICATION

(11) **EP 0 489 779 B1**
(45) Date of publication and mention of the grant of the patent: **28.01.1998**
(21) Application number: 90912700.3
(22) Date of filing: 25.07.1990
(51) Int. Cl.: A61K 31/58, A61K 31/56

(54) **OPHTHALMIC COMPOSITION**
OPHTHALMISCHE ZUBEREITUNG
COMPOSITION OPHTALMIQUE

(30) Priority: 28.08.1989 US 399351; 10.10.1989 US 419226
(43) Date of publication of application: 17.06.1992
(73) Proprietor: ALCON LABORATORIES, INC., Fort Worth Texas 76134-2099 (US)
(72) Inventor: CLARK, Abbot, F., Arlington, TX 76017 (US)
(74) Representative: Dowden, Marina
(86) International application number: US9004071
(87) International publication number: WO9103245

(56) References cited:
- EP-A- 0 088 462
- WO-A-87/02189
- Current Eye Research, vol. 8 (3), 1989, pages 317-320
- Endocrinology, vol. 119 (1), 1986, pages 375-380
- IOVS, vol. 34, 1993, page 925
- Biochemical Pharmacology, vol. 37 (3), 1988, pages 556-557

## Description

### Background of the Invention

### Field of the Invention

The present invention relates to the field of ophthalmology. More particularly, this invention relates to the treatment of inflamed ocular tissue.

### Description of Related Art

Many compounds classified as glucocorticoids, such as dexamethasone and prednisolone, are very effective in the treatment of inflamed tissues, but in certain patients, these compounds cause elevations in intraocular pressure. Patients who experience elevations in intraocular pressure when treated with glucocorticoids are generally referred to as "steroid responders." The elevations in intraocular pressure are of particular concern in patients who are already suffering from elevated intraocular pressures, such as glaucoma patients. Moreover, there is always a risk that the use of glucocorticoids in patients who have normal intraocular pressures will cause elevations in pressure that result in damage to ocular tissue. Since therapy with glucocorticoids is frequently long term (i.e., several days or more), there is potential for significant damage to ocular tissue as a result of prolonged elevations in intraocular pressure attributable to that therapy.

The following articles may be referenced for further background information concerning the well-recognized association between ophthalmic glucocorticoid therapy and elevations in intraocular pressure:
Kitazawa, "Increased Intraocular Pressure Induced by Corticosteroids," Am. J. Ophthal ., 82:492-493 (1976);
Cantrill, et al., "Comparison of In Vitro Potency of Corticosteroids with Ability to Raise Intraocular Pressure," Am. J. Ophthal., 79:1012-1016 (1975); and
Mindel, et al., "Comparative Ocular Pressure Elevation by Medrysone, Fluorometholone, and Dexamethasone Phosphate," Arch. Ophthal., 98:1577-1578 (1980).

One approach to solving the foregoing problems has been to search for compounds which are capable of alleviating ophthalmic inflammation without elevating intraocular pressure. The inventions described in commonly assigned U.S. Patent No. 4,686,214 (Boltralik) and copending U.S. Patent Application Serial No. 299,997 filed January 23, 1989 (Boltralik) represent two examples of this approach. Notwithstanding the success of the therapies described in the above-cited inventions, there continues to be a need for still further improvements in the treatment of ophthalmic inflammation, such as an improvement which would allow potent glucocorticoids to be utilized to treat inflamed ocular tissue without fear of elevating intraocular pressure.

### Summary of the Invention

A principal objective of the present invention is the provision of a medicament for the treatment of opthalmic inflammation which allows the potent anti-inflammatory activity of the glucocorticoids to be employed without fear of elevating intraocular pressure.

The foregoing objectives and other general objectives of the present invention are met by the provision of a medicament for ophthalmic inflammation wherein the elevations in intraocular pressure caused by glucocorticoids are substantially prevented. The present invention involves the combination of a first component, i.e. a glucocorticoid and a second component, i.e. an angiostatic steroid which prevents or antagonizes the intraocular pressure elevating effect of the glucocorticoid. This combination allows the intraocular pressure ("IOP") elevating effect of glucocorticoids to be eliminated without adversely affecting the anti-inflammatory activity of the glucocorticoids. Thus, the present invention makes it possible to employ the potent anti-inflammatory properties of the glucocorticoids without causing any significant elevations in intraocular pressure.

### Description of the Invention

The present invention is based on the combination of one or more potent glucocorticoids with one or more angiostatic steroids. For purposes of the present invention, the term "angiostatic steroids" means steroids and steroid metabolites which inhibit angiogenesis.

The present invention is based on the finding that angiostatic steroids somehow inhibit the IOP elevating effect of glucocorticoids. The mechanism by which angiostatic steroids prevent or antagonize the IOP elevating effect of glucocorticoids is not totally understood at this point. While applicant does not wish to be bound by any theory, one possible explanation is that these compounds interfere with the action of glucocorticoids on trapecular meshwork cells, thereby blocking or reversing the IOP elevating effect of the glucocorticoids.

The angiostatic steroids utilized in the present invention include all pharmaceutically acceptable steroids and steroid metabolites which inhibit angiogenesis. The preferred angiostatic steroids have been previously disclosed in PCT/US86/02189, and have the following formula: 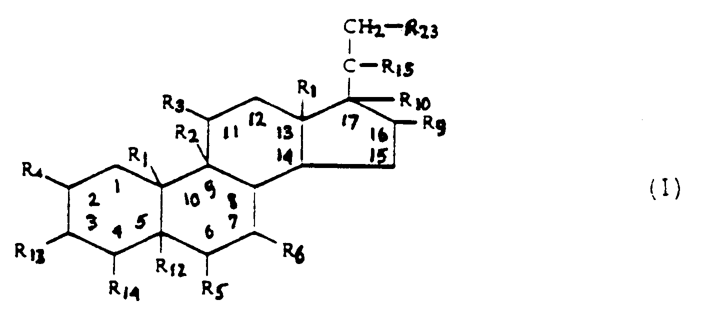 wherein:
- R₁: is β-CH₃ or β-CH₂H₅;
- R₂: is H or -Cl;
- R₃: is H, =O, -OH, -O-alkyl(C₁-C₁₂), -OC(=O)alkyl (C₁-C₁₂),- OC(=O)ARYL, -OC(=O)N(R)₂ or α-OC(=O)OR₇, wherein aryl is furyl, thienyl, pyrrolyl, or pyridyl and each of said moieties is optionally substituted with one or two (C₁-C₄)alkyl groups, or aryl is -(CH₂)_{f} phenyl wherein f is an integer from 0 to 2 and the phenyl ring is optionally substituted with 1 to 3 groups selected from chlorine: fluorine, bromine, alkyl (C₁-C₃), alkoxy (C₁-C₃), thioalkoxy-(C₁-C₃), Cl₃C-, F₃C-, -NH₂ and -NHCOCH₃ and R is hydrogen, alkyl (C₁-C₄), or phenyl and each R can be the same or different, and R₇ is aryl as herein defined, or alkyl (C₁-C₁₂); or
- R₂ and R₃: taken together are oxygen (-O-) bridging positions C-9 and C-11; or
- R₂ and R₃: taken together form a double bond between positions C-9 and C-11; or
- R₂: is α-F and R₃ is β-OH; or
- R₂: is α-Cl and R₃ is β-Cl; and
- R₄: is H, CH₃, Cl or F;
- R₅: is H, OH, F, Cl, Br, CH₃, phenyl, vinyl or allyl;
- R₆: is H or CH₃;
- R₉: is H, OH, CH₃, F or =CH₂;
- R₁₀: is H, OH, CH₃ or R₁₀ forms a second bond between positions C-16 and C-17;
- R₁₂: is -H or forms a double bond with R₁₄;
- R₁₃: is H, -OH, =O, -O-P(O)(OH)₂ or -O-C(=O)-(CH₂)ₜCOOH where t is an integer from 2 to 6:
- R₁₄: is H or forms a double bond with R₁₂;
- R₁₅: is =O or -OH; and
- R₂₃: with R₁₀ forms a cyclic phosphate as depicted by the following formula: 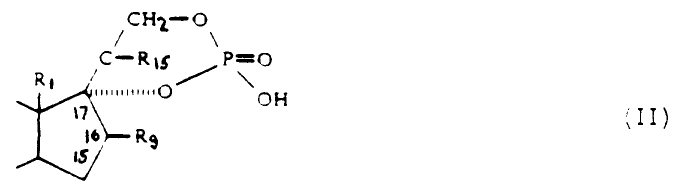 wherein R₁, R₉ and R₁₅ have the meaning defined above; or R₂₃ is -OH, O- C(=O)-R₁₁, -OP(O)-(OH)₂, or -O-C(=O)-(CH₂)ₜCOOH wherein t is an integer from 2 to 6; and R₁₁ is -Y-(CH₂)ₙ-X-(CH₂)ₘ-SO₃H, -Y'-(CH₂)p-X'-(CH₂)_{q}-NR₁₆R₁₇ or -Z(CH₂)ᵣQ, wherein Y is a bond or -O-; Y' is a bond, -O-, or -S-; each of X and X' is a bond, -CON(R₁₈)-, - N(R₁₈)CO-, -O-, -S-, -S(O)-, or -S(O₂)-; R₁₈ is hydrogen or alkyl (C₁-C₄); each of R₁₆ and R₁₇ is a lower alkyl group of from 1 to 4 carbon atoms optionally substituted with one hydroxyl or R₁₆ and R₁₇ taken together with the nitrogen atom to which each is attached forms a monocyclic heterocyclic selected from pyrrolidino, piperidino, morpholino, thiomorpholino, piperazino or N(lower)alkyl-piperazino wherein alkyl has 1 to 4 carbon atoms; n is an integer from 4 to 9; m is an integer from 1 to 5; p is an integer from 2 to 9: q is an integer from 1 to 5; wherein Z is a bond or -O-; r is an integer of from 2 to 9; and Q is one of the following:
(1) -R₁₉-CH₂COOH wherein R₁₉ is -S-, -S(O)-, -S(O)₂-, -SO₂N(R₂₀)-, or N(R₂₀)SO₂-; and R₂₀ is hydrogen or lower alkyl (C₁-C₄); with the proviso that the total number of carbon atoms in R₂₀ and (CH₂)ᵣ is not greater than 10; or
(2)-CO-COOH; or
(3) CON(R₂₁)CH(R₂₂)COOH wherein R₂₁ is H and R₂₂ is H, CH₃, -CH₂COOH, -CH₂CH₂COOH, -CH₂OH, -CH₂SH, -CH₂CH₂SCH₃, or -CH₂Ph-OH wherein Ph-OH is p-hydroxyphenyl; or R₂₁ is CH₃ and R₂₂ is H; or R₂₁ and R₂₂ taken together are -CH₂CH₂CH₂-; or -N(R₂₁)CH(R₂₂)COOH taken together is -NHCH₂CONHCH₂COOH,
with the proviso that except for the compound wherein R₁ is -CH₃, R₂ and R₃ taken together form a double bond between positions 9 and 11, R₄ and R₆ are hydrogen, R₁₂ and R₁₄ taken together form a double bond between positions 4 and 5, R₅ is α-F, R₉ is β-CH₃, R₁₀ is α-OH, R₁₃ and R₁₅ are =0 and R₂₃ is -OP(o)-(OH)₂, R₁₃ is =O only when R₂₃ with R₁₀ forms the above described cyclic phosphate, and pharmaceutically acceptable salts thereof.

Excepted from the compounds of Formula (I) is the compound 3,11β, 17α, 21-tetrahydroxy-5 pregnane-20-one (the 3-alpha, 5-beta; 3-alpha, 5-alpha; 3-beta, 5-alpha; and 3-beta, 5-beta isomers of tetrahydrocortisol) wherein;
R-₁₅ is =O; R₁₀ is αOH;
R₁ is CH₃; R₃ is βOH; R₂ is H; R₄ is H; R₁₃ is α or βOH; R₁₄ is H;
R₁₂ is α or βH; R₅ is H; R₆ is H; R₉ is H and R₂₃ is OH.

Unless specified otherwise, all substituent groups attached to the cyclopenta phenanthrene moiety of Formula (I) may be in either the alpha or beta position. Additionally, the above structures include all pharmaceutically acceptable salts of the angiostatic steroids. The use of the above-described angiostatic steroids to control intraocular pressure is described in applicant's commonly assigned U.S. Patent No. 4,876,250 issued October 24, 1989;

Preferred angiostatic steroids of the above formula include: 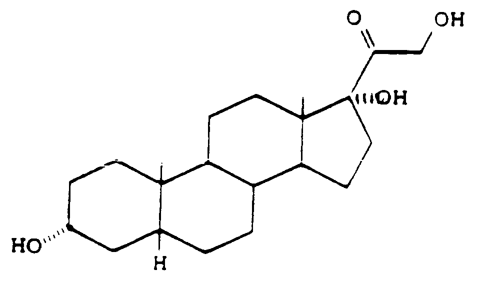
5β-pregnan-3α,17α, 21-triol-20-one, which is also known as tetrahydrocortexolone, and its pharmaceutically acceptable salts; 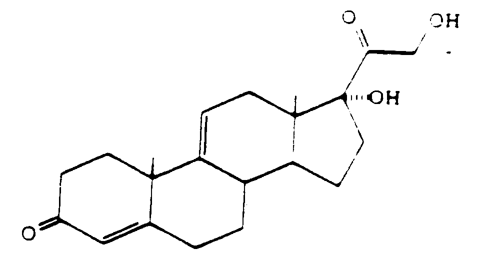
4,9(11)-pregnadien 17α,21-diol-3,20-dione, and its pharmaceutically acceptable salts; and 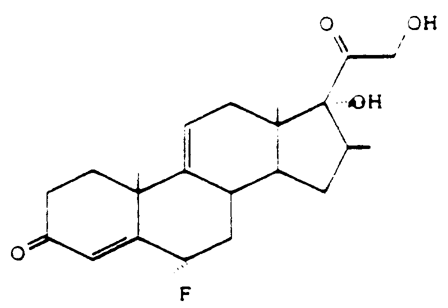
6 -fluoro-17α,21-dihydroxy-16β-methyl-pregan-4,9(11)-diene-3,20-dione, and its pharmaceutically acceptable salts.

Tetrahydrocortexolone is a particularly preferred angiostatic steroid. It is a known compound. It has a molecular weight of 350.5 and an empirical formula of C₂₁H₃₄O₄. The compound is commercially available and may, for example, be obtained from Sigma Chemical Company, P.O. Box 14508, St. Louis, MO 63178 or Steraloids, Inc., P.O. Box 310, Wilton, New Hampshire 03086.

The above-described compounds may exist in several stereoisomeric forms. Specifically, with regard to stereoisometry, for tetrahydrocortexolone it refers to relative positions of the hydroxyl and hydrogen groups at the 3,5 positions, as to whether or not they are above or below the plane of the ring structure. Alpha position refers to below the plane of the ring structure, and beta refers to above the ring structure. Thus, tetrahydrocortexolone may exist as 3-alpha, 5-beta; 3-alpha, 5-alpha; 3-beta, 5-alpha; and 3-beta, 5-beta. The preferred isomer for use in this invention is 3-alpha, 5-beta-tetrahydrocortexolone. The ring containing the 1-5 positions is referred to as the "A-ring".

The glucocorticoids which may be employed in the present invention include all pharmaceutically acceptable compounds which are effective in the treatment of inflamed ocular tissue. The preferred glucocorticoids include dexamethasone, fluorometholone, medrysone, betamethasone, triamcinolone, prednisone, prednisolone, hydrocortisone, and pharmaceutically acceptable salts thereof. Further examples of glucocorticoids include prednicarbate, deflazacort, halomethasone, tixocortol, prednylidene (21-diethylaminoacetate), prednival, paramethasone, methylprednisolone, meprednisone, mazipredone, isoflupredone, halopredone acetate, nalcinonide, formocortal, flurandrenolide, fluprednisolone, fluprednidine acetate, fluperolone acetate, fluocortolone, fluocortin butyl, fluocinonide, fluocinolone acetonide, flunisolide, flumethasone, fludrocortisone, fluclorinide, enoxolone, diflupregnate, diflucortolone, diflorasone diacetate, desoximetasone (desoxymethasone), desonide, descinolone, cortivazol, corticosterone, cortisone, cloprednol, clocortolone, clobetasone, clobetasol, chloroprednisone, cafestol, budesonide, beclomethasone, amcinonide, allopregnane acetonide, alclometasone, 21-acetoxypregnenolone, tralonide, diflorasone acetate, deacylcortivazol, RU-26988, budesonide, and deacylcortivazol oxetanone. All of the above-cited glucocorticoids are known compounds. Further information about the compounds may be found, for example, in The Merck Index, Eleventh Edition (1989), and the publications cited therein.

In accordance with the present invention, anti inflammatory, ophthalmic compositions containing one or more glucocorticoids and one or more angiostatic steroids are provided. The compositions will contain one or more glucocorticoids in an anti-inflammatory effective amount and will contain one or more angiostatic steroids in an amount effective to inhibit the IOP elevating effect of the glucocorticoids. The amount of each component will depend on various factors, such as the relative tendency of certain glucocorticoids to cause IOP elevations, the severity and type of ocular inflammation being treated, the estimated duration of the treatment, and so on. In general, the ratio of the amount of glucocorticoid to the amount of angiostatic steroid on a weight to weight basis will be in the range of 10:1 to 1:20. The concentration of the glucocorticoid component will typically be in the range of about 0.01% to about 2.0% by weight. The concentration of the angiostatic steroid component will typically be in the range of about 0.05% to about 5.0% by weight.

The above-described active ingredients may be incorporated into various types of ophthalmic formulations for delivery to the eye. For example, the active ingredients nay be combined with ophthalmologically acceptable preservatives, surfactants, viscosity enhancers, buffers, toxicity agents and water to form an aqueous, sterile ophthalmic suspension. In order to prepare sterile ophthalmic ointment formulations, the active ingredients are combined with a preservative in an appropriate vehicle, such as mineral oil, liquid lanolin, or white petrolatum. Sterile ophthalmic gel formulations may be prepared by suspending the active ingredient in a hydrophilic base prepared from the combination of Carbopol-940 (a carboxy vinyl polymer available from the B.F. Goodrich Company) according to published formulations for analogous ophthalmic preparations; preservatives and tonicity agents can also be incorporated. The specific type of formulation selected will depend on various factors, such as the severity and type of ophthalmic inflammation being treated, and dosage frequency. Ophthalmic solutions, suspensions, ointments and gels are the preferred dosage forms, and topical application to the inflamed ocular tissue is the preferred route of administration.

The following Example is presented to illustrate further the compositions of the present invention.

### Example

The following formulation is representative of the antiinflammatory compositions of the present invention.

| Ingredient | Amount (wt.%) |
|---|---|
| Tetrahydrocortexolone | 1.0 |
| Dexamethasone | 0.1 |
| Tyloxapol | 0.01 to 0.05 |
| HPMC | 0.5 |
| Benzalkonium chloride | 0.01 |
| Sodium chloride | 0.8 |
| Edetate Disodium | 0.01 |
| NaOH/HCl | q.s. pH 7.4 |
| Purified water | q.s. 100 mL |

The above formulation is prepared by first placing a portion of the purified water into a beaker and heating to 90°C. The hydroxypropylmethylcellulose (HPMC) is then added to the heated water and mixed by means of vigorous vortex stirring until all of the HPMC is dispersed. The resulting mixture is then allowed to cool while undergoing mixing in order to hydrate the HPMC. The resulting solution is then sterilized by means of autoclaving in a vessel having a liquid inlet and a hydrophobic, sterile air vent filter.

The sodium chloride and the edetate disodium are then added to a second portion of the purified water and dissolved. The benzalkonium chloride is then added to the solution, and the pH of the solution is adjusted to 7.4 with 0.1M NaOH/HCl. The solution is then sterilized by means of filtration.

The tetrahydrocortexolone and dexamethasone are sterilized by either dry heat or ethylene oxide. If ethylene oxide sterilization is selected, aeration for at least 72 hours at 50°C. is necessary. The sterilized THS and dexamethasone are weighed aseptically and placed into a pressurized ballmill container. The tyloxapol, in sterilized aqueous solution form, is then added to the ballmill container. Sterilized glass balls are then added to the container and the contents of the container are milled aseptically at 225 rpm for 16 hours, or until all particles are in the range of approximately 5 micrometers.

Under aseptic conditions, the micronized drug suspension formed by means of the preceding step is then poured into the HPMC solution with mixing. The ballmill container and balls contained therein are then rinsed with a portion of the solution containing the sodium chloride, the edetate disodium and benzalkonium chloride. The rinse is then added aseptically to the HPMC solution. The final volume of the solution is then adjusted with purified water and, if necessary, the pH of the solution is adjusted to pH 7.4 with NaOH/HCl.

The present invention comprises the application of an effective amount of the above-described compositions to the eye. The dosage regimen utilized will depend on the severity and type of inflammation being treated, as well as various clinical factors, such as, the patient's age, sex, weight and medical history. In general, the above-described compositions may be topically applied, for example, as drops to the upper globe, or as a 0.5-1 cm strip of ointment or gel to the lower conjunctival sac of the eye. Suspensions will generally be applied 1 to 4 times daily, while ointments or gels will generally be applied once or twice daily. The application of sustained release formulations (e.g., polymer based gels) once daily at bedtime will be preferred in some conditions. Intraocular routes of administration, such as injections or instillations in conjunction with intraocular surgery, are also contemplated.

## Claims

1. Use of a mixture of a glucocorticoid and an angiostatic steroid for the preparation of a pharmaceutical composition for the treatment of ophthalmic inflammation wherein the glucocorticoid is present in an amount to cause an anti-inflammatory effect and the angiostatic steroid is present in an amount which is sufficient to inhibit the glucocorticoid from elevating the intraocular pressure for the patient.

2. Use of a mixture of a glucocorticoid and an angiostatic steroid for the preparation of a pharmaceutical composition according to Claim 1, wherein the angiostatic steroid has the following formula: 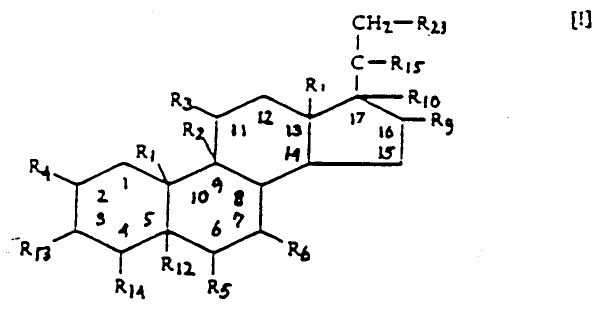 wherein:
R₁ is β-CH₃ or β-CH₂H₅;
R₂ is H or -Cl;
R₃ is H, =O, -OH, -O-alkyl(C₁-C₁₂), -OC(=O)alkyl(C₁-C₁₂), - OC(=O)aryl, -OC(=O)N(R)₂ or α-OC(=O)OR₇, wherein aryl is furyl, thienyl, pyrrolyl, or pyridyl and each of said moieties is optionally substituted with one or two (C₁-C₄)alkyl groups, or aryl is -(CH₂)_{f} phenyl wherein f is an integer from 0 to 2 and the phenyl ring is optionally substituted with 1 to 3 groups selected from chlorine, fluorine, bromine, alkyl (C₁-C₃), alkoxy (C₁-C₃), thioalkoxy-(C₁-C₃), Cl₃C-, F₃C-, -NH₂ and -NHCOCH₃ and R is hydrogen, alkyl (C₁-C₄), or phenyl and each R can be the same or different, and R₇ is aryl as herein defined, or alkyl (C₁-C₁₂); or
R₂ and R₃ taken together are oxygen (-O-) bridging positions C-9 and C-11; or
R₂ and R₃ taken together form a double bond between positions C-9 and C-11; or
R₂ is α-F and R₃ is β-OH; or
R₂ is α-Cl and R₃ is β-Cl; and
R₄ is H, CH₃, Cl or F;
R₅ is H, OH, F, Cl, Br, CH₃, phenyl, vinyl or allyl;
R₆ is H or CH₃;
R₉ is H, OH, CH₃, F or =CH₂;
R₁₀ is H, OH, CH₃ or R₁₀ forms a second bond between positions C-16 and C-17;
R₁₂ is -H or forms a double bond with R₁₄;
R₁₃ is H, -OH, =O, -O-P(O)(OH)₂ or -O-C(=O)-(CH₂)ₜCOOH where t is an integer from 2 to 6;
R₁₄ is H or forms a double bond with R₁₂;
R₁₅ is =O or -OH; and
R₂₃ with R₁₀ forms a cyclic phosphate as depicted by the following formula: 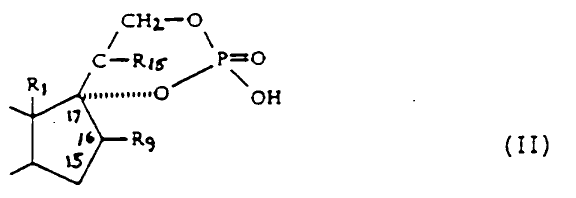 wherein R₁, R₉ and R₁₅ have the meaning defined above; or
R₂₃ is -OH, O-C(=O)-R₁₁, -OP(O)-(OH)₂, or -O-C(=O)-(CH₂)ₜCOOH wherein t is an integer from 2 to 6; and R₁₁ is -Y-(CH₂)ₙ-X-(CH₂)ₘ-SO₃H, -Y'-(CH₂)p-X'-(CH₂)_{q}-NR₁₆R₁₇ or - Z(CH₂)ᵣQ, wherein Y is a bond or -O-; Y' is a bond, -O-, or -S-; each of X and X' is a bond, -CON(R₁₈)-, -N(R₁₈)CO-, -O-, -S-, -S(O)-, or -S(O₂)-; R₁₈ is hydrogen or alkyl (C₁-C₄); each of R₁₆ and R₁₇ is a lower alkyl group of from 1 to 4 carbon atoms optionally substituted with one hydroxyl or R₁₆ and R₁₇ taken together with the nitrogen atom to which each is attached forms a monocyclic heterocyclic selected from pyrrolidino, piperidino, morpholino, thiomorpholino, piperazino or N(lower)alkyl-piperazino wherein alkyl has 1 to 4 carbon atoms; n is an integer from 4 to 9; m is an integer from 1 to 5; p is an integer from 2 to 9: q is an integer from 1 to 5; Z is a bond or -O-; r is an integer of from 2 to 9; and Q is one of the following:
(1) -R₁₉-CH₂COOH wherein R₁₉ is -S-, -S(O)-, -S(O)₂-, -SO₂N(R₂₀)-, or N(R₂₀)SO₂-; and R₂₀ is hydrogen or lower alkyl (C₁-C₄); with the proviso that the total number of carbon atoms in R₂₀ and (CH₂)ᵣ is not greater than 10; or
(2)-CO-COOH; or
(3) CON(R₂₁)CH(R₂₂)COOH wherein R₂₁ is H and R₂₂ is H, CH₃, - CH₂COOH, -CH₂CH₂COOH, -CH₂OH, -CH₂SH, -CH₂CH₂SCH₃, or -CH₂Ph-OH wherein Ph-OH is p-hydroxyphenyl; or R₂₁ is CH₃ and R₂₂ is H; or R₂₁ and R₂₂ taken together are -CH₂CH₂CH₂-; or -N(R₂₁)CH(R₂₂)COOH taken together is -NHCH₂CONHCH₂COOH.
with the proviso that except for the compound wherein R₁ is -CH₃, R₂ and R₃ taken together form a double bond between positions 9 and 11, R₄ and R₆ are hydrogen, R₁₂ and R₁₄ taken together form a double bond between positions 4 and 5, R₅ is α-F, R₉ is β-CH₃, R₁₀ is α-OH, R₁₃ and R₁₅ are =0 and R₂₃ is -OP(o)-(OH)₂, R₁₃ is =O only when R₂₃ with R₁₀ forms the above described cyclic phosphate; and excluding the compounds wherein R₁₅ is 0; R₁₀ is OH; R₁ is CH₃; R₃ is βOH; R₂ is H; R₄ is H; R₁₃ is α or β-OH; R₁₄ is H; R₁₂ is α or βH; R₉ is H; R₆ is H; R₉ is H and R₂₃ is OH;
or a pharmaceutically acceptable salt thereof.

3. Use of a mixture of a glucocorticoid and an angiostatic steroid for the preparation of a pharmaceutical composition of Claim 1, wherein the angiostatic steroid comprises tetrahydrocortexolone or a pharmaceutically acceptable salt thereof.

4. Use of a mixture of a glucocorticoid and an angiostatic steroid for the preparation of a pharmaceutical composition of Claim 1, wherein the angiostatic steroid comprises 6α-fluoro-17α,21-dihydroxy-16β-methyl-pregna-4,9(11)-diene-3,20-dione, or a pharmaceutically acceptable salt thereof.

5. Use of a mixture of a glucocorticoid and an angiostatic steroid for the preparation of a pharmaceutical composition of Claim 1, wherein the angiostatic steroid comprises 4,9(11)-pregnadien 17α,21-diol-3,20-dione, or a pharmaceutically acceptable salt thereof.

6. Use of a mixture of a glucocorticoid and an angiostatic steroid for the preparation of a pharmaceutical composition of Claim 1, wherein the glucocorticoid is selected from dexamethasone, fluorometholone, medrysone, betamethasone, triamcinolone, prednisone, prednisolone, hydrocortisone, and pharmaceutically acceptable salts thereof.

7. Use of a mixture of a glucocorticoid and an angiostatic steroid for the preparation of a pharmaceutical composition of Claim 1, wherein the ratio of the amount of the glucocorticoid to the amount of an angiostatic steroid on a weight to weight basis in the range of 10:1 to 1:20.

8. Use of a mixture of a glucocorticoid and an angiostatic steroid for the preparation of a pharmaceutical composition of Claim 1, wherein the glucocorticoid is contained in the composition in an amount in the range of 0.01% to 2.0% by weight.

9. Use of a mixture of a glucocorticoid and an angiostatic steroid for the preparation of a pharmaceutical composition of Claim 1, wherein the angiostatic steroid is contained in the composition in an amount in the range of 0.05% to 5.0% by weight.

## Patentansprüche

1. Verwendung eines Gemisches aus einem Glucocorticoid und einem angiostatischen Steroid zur Herstellung eines Arzneimittels zur Behandlung von Augenentzündungen, wobei das Glucocorticoid in einer Menge vorhanden ist, daß ein antiinflammatorischer Effekt bewirkt wird und das angiostatische Steroid in einer Menge vorhanden ist, die ausreichend ist, die Erhöhung des intraokularen Drucks des Patienten durch das Glucocorticoid zu hemmen.

2. Verwendung eines Gemisches aus einem Glucocorticoid und einem angiostatischen Steroid zur Herstellung eines Arzneimittels nach Anspruch 1, dadurch **gekennzeichnet,** daß das angiostatische Steroid die folgende Formel 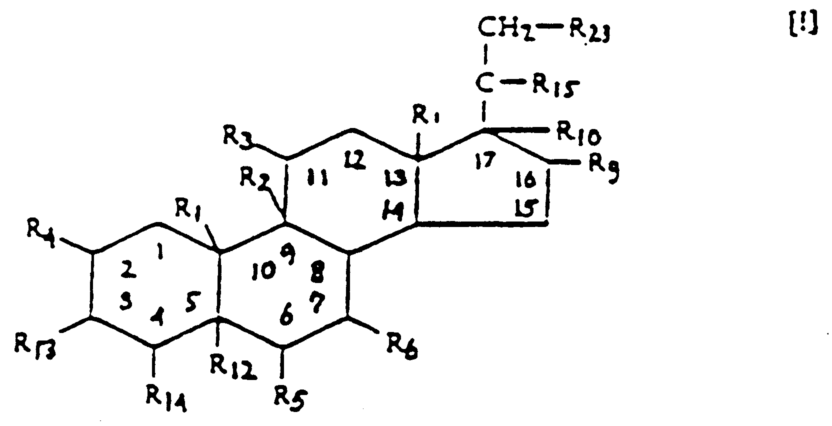 aufweist, worin:
R₁ für β-CH₃ oder β-CH₂H₅ steht;
R₂ für H oder -Cl steht;
R₃ für H, =O, -OH, -O-Alkyl(C₁-C₁₂), -OC(=O)Alkyl(C₁-C₁₂), -OC(=O)Aryl, -OC(=O)N(R)₂ oder α-OC(=O)OR₇, wobei Aryl die Bedeutung Furyl, Thienyl, Pyrrolyl oder Pyridyl hat, steht, und jede der genannten Gruppierungen ggf. durch eine oder zwei (C₁-C₄)Alkylgruppen substituiert ist, oder die Arylgruppe -(CH₂)_{f}-Phenyl ist, worin f eine ganze Zahl von 0 bis 2 ist und wobei der Phenylring ggf. mit 1 bis 3 Gruppen, ausgewählt aus Chlor, Fluor, Brom, Alkyl(C₁-C₃), Alkoxy(C₁-C₃), Thioalkoxy-(C₁-C₃), Cl₃C-, F₃C-, -NH₂ und -NHCOCH₃, substituiert ist und R für Wasserstoff, Alkyl(C₁-C₄) oder Phenyl steht und jedes R gleich oder verschieden sein kann und wobei R₇ für Aryl, wie hierin definiert, oder Alkyl(C₁-C₁₂) steht; oder R₂ und R₃ miteinander genommen Sauerstoff(-O-)-überbrückende Positionen C-9 und C-11 sind; oder
R₂ und R₃ miteinander eine Doppelbindung zwischen den Positionen C-9 und C-11 bilden; oder
R₂ für α-F steht und R₃ für β-OH steht; oder
R₂ für α-Cl und R₃ für β-Cl steht; und
R₄ für H, CH₃, Cl oder F steht;
R₅ für H, OH, F, Cl, Br, CH₃, Phenyl, Vinyl oder Allyl steht;
R₆ für H oder CH₃ steht;
R₉ für H, OH, CH₃, F oder =CH₂ steht;
R₁₀ für H, OH, CH₃ steht oder R₁₀ eine zweite Bindung zwischen den Positionen C-16 und C-17 bildet;
R₁₂ für -H steht oder eine Doppelbindung mit R₁₄ bildet;
R₁₃ für H, -OH, =O, -O-P(O)(OH)₂ oder -O-C(=O)-(CH₂)ₜCOOH steht, wobei t eine ganze zahl von 2 bis 6 ist;
R₁₄ für H steht oder eine Doppelbindung mit R₁₂ bildet;
R₁₅ für =O oder -OH steht; und
R₂₃ mit R₁₀ ein cyclisches Phosphat, wie durch die folgende Formel: 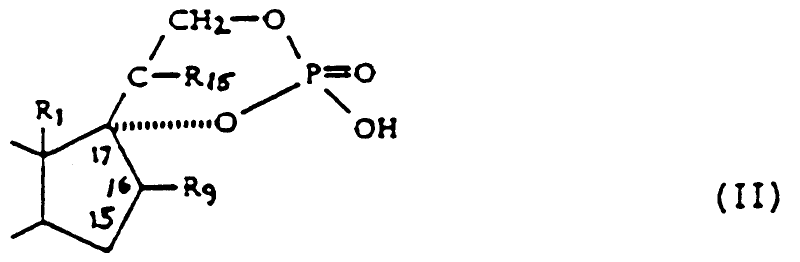 angegeben, bildet, worin R₁, R₉ und R₁₅ die oben angegebenen Definitionen haben,; oder R₂₃ für -OH, O-C(=O)-R₁₁, -OP(O)-(OH)₂ oder -O-C(=O)-(CH₂)ₜCOOH steht, wobei t eine ganze Zahl von 2 bis 6 ist; und R₁₁ für -Y-(CH₂)ₙ-X-(CH₂)ₘ-SO₃H, -Y'-(CH₂)ₚ-X'-(CH₂)_{q}-NR₁₆R₁₇ oder -Z(CH₂)ᵣQ steht, wobei Y eine Bindung oder -O- ist; Y' eine Bindung, -O- oder -S- bedeutet; X und X' jeweils eine Bindung -CON(R₁₈)-, -N(R₁₈)CO-, -O-, -S-, -S(O)- oder -S(O₂)- ist; R₁₈ für Wasserstoff oder Alkyl(C₁-C₄) steht; R₁₆ und R₁₇ jeweils eine Niedrigalkylgruppe mit 1 bis 4 Kohlenstoffatomen, die ggf. mit einer Hydroxylgruppe substituiert ist, steht; oder R₁₆ und R₁₇ zusammen mit dem Stickstoffatom, an das sie angefügt sind, einen monocyclischen heterocyclischen, ausgewählt aus Pyrrolidino, Piperidino, Morpholino, Thiomorpholino, Piperazino oder N(Niedrig)alkylpiperazino, bilden, wobei das Alkyl 1 bis 4 Kohlenstoffatome hat; n eine ganze Zahl von 4 bis 9 ist; m eine ganze Zahl von 1 bis 5 ist; p eine ganze Zahl von 2 bis 9 ist; q eine ganze Zahl von 1 bis 5 ist; Z eine Bindung oder -O- ist; r eine ganze Zahl von 2 bis 9 ist; und Q eine der folgenden Bedeutungen hat:
(1) -R₁₉-CH₂COOH, wobei R₁₉ für -S-, -S(O)-, -S(O)₂-, -SO₂N(R₂₀)- oder N(R₂₀)SO₂- steht; und R₂₀ für Wasserstoff oder Niedrigalkyl(C₁-C₄) steht; mit der Maßgabe, daß die Gesamtzahl der Kohlenstoffatome in R₂₀ und (CH₂)ᵣ nicht größer als 10 ist; oder
(2) -CO-COOH; oder
(3) CON(R₂₁)CH(R₂₂)COOH, wobei R₂₁ für H steht und R₂₂ für H, CH₃, -CH₂COOH, -CH₂CH₂COOH, -CH₂OH, -CH₂SH, -CH₂CH₂SCH₃ oder -CH₂Ph-OH, wobei Ph-OH p-Hydroxyphenyl ist; oder wobei R₂₁ für CH₃ steht und R₂₂ für H steht; oder R₂₁ und R₂₂ miteinander genommen -CH₂CH₂CH₂- sind; oder
-N(R₂₁)CH(R₂₂)COOH zusammengenommen -NHCH₂CONHCH₂COOH ist, mit der Maßgabe, daß ausgenommen die Verbindung, bei der R₁ für CH₃ steht, R₂ und R₃ miteinander genommen eine Doppelbindung zwischen den Positionen 9 und 11 bilden, R₄ und R₆ Wasserstoff sind, R₁₂ und R₁₄ miteinander eine Doppelbindung zwischen den Positionen 4 und 5 bilden, R₅ für α-F steht, R₉ für β-CH₃, R₁₀ für α-OH steht, R₁₃ und R₁₄ =O sind und R₂₃ für -OP(o)-(OH)₂ steht, R₁₃ nur dann die Bedeutung =O hat, wenn R₂₃ mit R₁₀ das oben beschriebene cyclische Phosphat bilden; und mit Ausschluß der Verbindungen, bei denen R₁₅ für O steht; R₁₀ für OH steht; R₁ für CH₃ steht; R₃ für β-OH steht; R₂ für H steht; R₄ für H steht; R₁₃ für α- oder β-OH steht; R₁₄ für H steht; R₁₂ für α- oder β-H steht; R₅ für H steht; R₆ für H steht; R₉ für H steht und R₂₃ für OH steht;
oder ein pharmazeutisch annehmbares Salz davon.

3. Verwendung eines Gemisches aus einem Glucocorticoid und einem angiostatischen Steroid zur Herstellung eines Arzneimittels nach Anspruch 1, dadurch **gekennzeichnet,** daß das angiostatische Steroid Tetrahydrocortexolon oder ein pharmazeutisch annehmbares Salz davon ist.

4. Verwendung eines Gemisches aus einem Glucocorticoid und einem angiostatischen Steroid zur Herstellung eines Arzneimittels nach Anspruch 1, dadurch **gekennzeichnet,** daß das angiostatische Steroid 6α-Fluor-17α,21-dihydroxy-16β-methylpregna-4,9(11)-dien-3,20-dion oder ein pharmazeutisch annehmbares Salz davon ist.

5. Verwendung eines Gemisches aus einem Glucocorticoid und einem angiostatischen Steroid zur Herstellung eines Arzneimittels nach Anspruch 1, dadurch **gekennzeichnet,** daß das angiostatische Steroid 4,9(11)-Pregnadien-17α,21-diol-3,20-dion oder ein pharmazeutisch annehmbares Salz davon ist.

6. Verwendung eines Gemisches aus einem Glucocorticoid und einem angiostatischen Steroid zur Herstellung eines Arzneimittels nach Anspruch 1, dadurch **gekennzeichnet,** daß das Glucocorticoid aus Dexamethason, Fluormetholon, Medryson, Betamethason, Triamcinolon, Prednison, Prednisolon, Hydrocortison und pharmazeutisch annehmbaren Salzen davon ausgewählt ist.

7. Verwendung eines Gemisches aus einem Glucocorticoid und einem angiostatischen Steroid zur Herstellung eines Arzneimittels nach Anspruch 1, dadurch **gekennzeichnet,** daß das Verhältnis der Menge des Glucocorticoids zu der Menge des angiostatischen Steroids auf Gewicht-Gewichts-Basis im Bereich von 10:1 bis 1:20 liegt.

8. Verwendung eines Gemisches aus einem Glucocorticoid und einem angiostatischen Steroid zur Herstellung eines Arzneimittels nach Anspruch 1, dadurch **gekennzeichnet,** daß das Glucocorticoid in der Zubereitung in einer Menge im Bereich von 0,01 bis 2,0 Gew.-% enthalten ist.

9. Verwendung eines Gemisches aus einem Glucocorticoid und einem angiostatischen Steroid zur Herstellung eines Arzneimittels nach Anspruch 1, dadurch **gekennzeichnet,** daß das angiostatische Steroid in der Zubereitung in einer Menge im Bereich von 0,05 bis 5,0 Gew.-% enthalten ist.

## Revendications

1. Utilisation d'un mélange d'un glucocorticoïde et d'un stéroïde angiostatique pour la préparation d'une composition pharmaceutique pour le traitement d'inflammations ophtalmiques, dans laquelle le glucocorticoïde est présent en une quantité permettant d'obtenir un effet anti-inflammatoire et le stéroïde angiostatique est présent en une quantité suffisante pour empêcher le glucocorticoïde d'élever la pression intra-oculaire du patient.

2. Utilisation d'un mélange d'un glucocorticoïde et d'un stéroïde angiostatique pour la préparation d'une composition pharmaceutique selon la revendication 1, dans laquelle le stéroïde angiostatique répond à la formule ci-après: 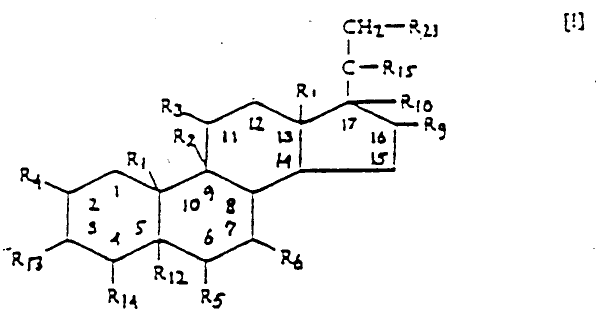 dans laquelle:
R₁ représente un groupe β-CH₃ ou un groupe β-CH₂H₅;
R₂ représente H ou -Cl;
R₃ représente H, =O, -OH, un groupe -O-alkyle en C₁-C₁₂, un groupe -OC(=O)alkyle en C₁-C₁₂, un groupe -OC(=O)aryle, un groupe -OC(=O)N(R)₂ ou un groupe α-OC(=O)OR₇, le groupe aryle représentant un groupe furyle, un groupe thiényle, un groupe pyrrolyle ou un groupe pyridyle, chacune desdites fractions étant éventuellement substituée par 1 ou 2 groupes alkyle en C₁-C₄, ou bien le groupe aryle représente un groupe -(CH₂)_{f}-phényle où f représente un entier de 0 à 2 et le noyau phényle est éventuellement substitué par 1 à 3 groupes choisis parmi un atome de chlore, un atome de fluor, un atome de brome, un groupe alkyle en C₁-C₃, un groupe alcoxy en C₁-C₃, un groupe thioalcoxy en C₁-C₃, un groupe Cl₃C-, un groupe F₃C-, un groupe -NH₂ et un groupe -NHCOCH₃, et R représente un atome d'hydrogène, un groupe alkyle en C₁-C₄ ou encore un groupe phényle, chacun des radicaux R pouvant être identique ou différent, et R₇ représente un groupe aryle tel que défini ci-dessus ou encore un groupe alkyle en C₁-C₁₂; ou bien
R₂ et R₃ pris ensemble forment un pont d'oxygène (-O-) entre les positions C-9 et C-11; ou bien
R₂ et R₃ pris ensemble forment une liaison double entre les positions C-9 et C-11; ou encore
R₂ représente α-F et R₃ représente β-OH; ou bien
R₂ représente α-Cl et R₃ représente β-Cl; et
R₄ représente H, CH₃, Cl ou F;
R₅ représente H, OH, F, Cl, Br, CH₃, un groupe phényle, un groupe vinyle ou un groupe allyle;
R₆ représente H ou CH₃;
R₉ représente H, OH, CH₃, F ou =CH₂;
R₁₀ représente H, OH, CH₃ ou bien R₁₀ forme une seconde liaison entre les positions C-16 et C-17;
R₁₂ représente -H ou forme une liaison double avec R₁₄;
R₁₃ représente H, -OH, =O, un groupe -O-P(O)(OH)₂ ou un groupe -O-C(=O)-(CH₂)ₜCOOH où t représente un entier de 2 à 6;
R₁₄ représente H ou forme une liaison double avec R₁₂;
R₁₅ représente =O ou -OH; et
R₂₃ forme avec R₁₀ un groupe phosphate cyclique répondant à la formule ci-après 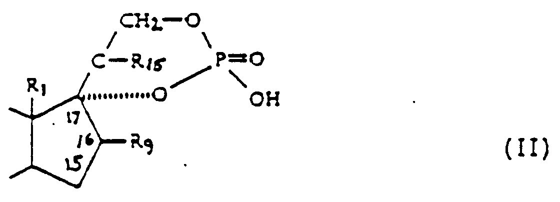 dans laquelle R₁, R₉ et R₁₅ ont la signification définie ci-dessus; ou bien
R₂₃ représente -OH, un groupe O-C(=O)-R₁₁, un groupe -OP(O)-(OH)₂ ou un groupe -O-C(=O)-(CH₂)ₜCOOH où t représente un entier de 2 à 6; et R₁₁ représente un groupe -Y-(CH₂)ₙ-X-(CH₂)ₘ-SO₃H, un groupe -Y'-(CH₂)ₚ-X'-(CH₂)_{q}-NR₁₆R₁₇ ou encore un groupe -Z(CH₂)ᵣQ où Y représente une liaison ou -O-; Y' représente une liaison, -O- ou -S-; chacun des radicaux X et X' représente une liaison, un groupe -CON(R₁₈)-, un groupe -N(R₁₈)CO-, -O-, -S-, -S(O)- ou -S(O₂)-; R₁₈ représente un atome d'hydrogène ou un groupe alkyle en C₁-C₄; chacun des radicaux R₁₆ et R₁₇ représente un groupe alkyle inférieur contenant de 1 à 4 atomes de carbone, éventuellement substitué par un groupe hydroxyle; ou bien R₁₆ et R₁₇ pris ensemble avec l'atome d'azote auquel chacun est fixé forment un radical hétérocyclique/monocyclique choisi parmi un groupe pyrrolidino, un groupe pipéridino, un groupe morpholino, un groupe thiomorpholino, un groupe pipérazino ou un groupe N-alkyl(inférieur)pipérazino dans lequel le groupe alkyle contient de 1 à 4 atomes de carbone; n représente un entier de 4 à 9; m représente un entier de 1 à 5; p représente un entier de 2 à 9; q représente un entier de 1 à 5; Z représente une liaison ou -O-; r représente un entier de 2 à 9; et Q représente un des groupes ci-après:
(1) un groupe -R₁₉-CH₂COOH dans lequel R₁₉ représente -S-, -S(O)-, -S(O)₂-, un groupe -SO₂N(R₂₀)- ou un groupe N(R₂₀)SO₂-; et R₂₀ représente un atome d'hydrogène ou un groupe alkyle inférieur en C₁-C₄;
avec cette réserve que le nombre total des atomes de carbone dans R₂₀ et dans (CH₂)ᵣ n'est pas supérieur à 10; ou bien
(2) un groupe -CO-COOH; ou encore
(3) un groupe CON(R₂₁)CH(R₂₂)COOH où R₂₁ représente H et R₂₂ représente H, CH₃, un groupe CH₂COOH, un groupe -CH₂CH₂COOH, un groupe -CH₂OH, un groupe -CH₂SH, un groupe -CH₂CH₂SCH₃ ou un groupe -CH₂Ph-OH où Ph-OH représente un groupe p-hydroxyphényle; ou encore R₂₁ représente CH₃ et R₂₂ représente H; ou bien R₂₁ et R₂₂ pris ensemble représentent un groupe -CH₂CH₂CH₂-; ou encore un groupe -N(R₂₁)CH(R₂₂)COOH dans lequel R₂₁ et R₂₂ pris ensemble représentent un groupe -NHCH₂CONHCH₂COOH,
avec cette réserve qu'à l'exception du composé dans lequel R₁ représente -CH₃, R₂ et R₃ pris ensemble forment une liaison double entre les positions 9 et 11, R₄ et R₆ représentent un atome d'hydrogène, R₁₂ et R₁₄ pris ensemble forment une liaison double entre les positions 4 et 5, R₅ représente α-F, R₉ représente β-CH₃, R₁₀ représente α-OH, R₁₃ et R₁₅ représentent =O et R₂₃ représente un groupe -OP(O)-(OH)₂, R₁₃ représente =O uniquement lorsque R₂₃ forme avec R₁₀ le phosphate cyclique décrit ci-dessus, et à l'exclusion des composés dans lesquels R₁₅ représente O, R₁₀ représente OH, R₁ représente CH₃, R₃ représente β-OH, R₂ représente H, R₄ représente H, R₁₃ représente α- ou β-OH, R₁₄ représente H, R₁₂ représente α- ou β-H, R₅ représente H, R₆ représente H, R₉ représente H et R₂₃ représente OH;
ou encore d'un de leurs sels pharmaceutiquement acceptables.

3. Utilisation d'un mélange d'un glucocorticoïde et d'un stéroïde angiostatique pour la préparation d'une composition pharmaceutique selon la revendication 1, dans laquelle le stéroïde angiostatique comprend la tétrahydrocortexolone ou un de ses sels pharmaceutiquement acceptables.

4. Utilisation d'un mélange d'un glucocorticoïde et d'un stéroïde angioscatique pour la préparation d'une composition pharmaceutique selon la revendication 1, dans laquelle le stéroïde angiostatique comprend la 6α-fluoro-17α,21-dihydroxy-16β-méthyl-pregna-4,9(11)-diène-3,20-dione ou un de ses sels pharmaceutiquement acceptables.

5. Utilisation d'un mélange d'un glucocorticoïde et d'un stéroïde angiostatique pour la préparation d'une composition pharmaceutique selon la revendication 1, dans laquelle le stéroïde angiostatique comprend la 4,9(11)-pregnadiène-17α,21-diol-3,20-dione ou un de ses sels pharmaceutiquement acceptables.

6. Utilisation d'un mélange d'un glucocorticoïde et d'un stéroïde angiostatique pour la préparation d'une composition pharmaceutique selon la revendication 1, dans laquelle le glucocorticoïde est choisi parmi la dexaméthasone, la fluorométholone, la médrysone, la bêtaméthasone, la triamcinolone, la prednisone, la prednisolone, l'hydrocortisone, et leurs sels pharmaceutiquement acceptables.

7. Utilisation d'un mélange d'un glucocorticoïde et d'un stéroïde angiostatique pour la préparation d'une composition pharmaceutique selon la revendication 1, dans laquelle le rapport de la quantité du glucocorticoïde à la quantité d'un stéroïde angiostatique sur une base en poids/poids se situe dans le domaine de 10:1 à 1:20.

8. Utilisation d'un mélange d'un glucocorticoïde et d'un stéroïde angiostatique pour la préparation d'une composition pharmaceutique selon la revendication 1, dans laquelle le glucocorticoïde est contenu dans la composition en une quantité dans le domaine de 0,01% à 2,0% en poids.

9. Utilisation d'un mélange d'un glucocorticoïde et d'un stéroïde angiostatique pour la préparation d'une composition pharmaceutique selon la revendication 1, dans laquelle le stéroïde angiostatique est contenu dans la composition en une quantité dans le domaine de 0,05% à 5,0% en poids.
